# EUROPEAN PATENT APPLICATION

(11) **EP 1 357 216 A1**
(43) Date of publication of application: **29.10.2003**
(21) Application number: 01938623.4
(22) Date of filing: 13.06.2001
(51) Int. Cl.: D04H 3/16, D04H 3/00, A61F 13/53

(54) **SPUNBONDED NONWOVEN FABRIC AND ABSORBENT ARTICLE**

(30) Priority: 13.06.2000 JP 2000176508; 23.06.2000 JP 2000188726; 26.07.2000 JP 2000225657
(71) Applicant: Idemitsu Unitech Co., Ltd., Tokyo 112-0002 (JP)
(72) Inventor: ISHIKAWA, Masahide, Kujukuri-machi, Sanbu-gun, Chiba 283-010 (JP); KURAHASHI, Akihiko, Kujukuri-machi, Sanbu-gun Chiba 283-0101 (JP)
(74) Representative: Hucker, Charlotte Jane
(86) International application number: JP0104984
(87) International publication number: WO01096641

(57) **Abstract**

A spunbonded nonwoven fabric which is made of a polyolefin resin and has an average fiber diameter of 5 to 60 µm, area weight of 5 to 200 g/m², and a coefficient of static friction of 0.1 to 0.4 ; or a spunbonded nonwoven fabric which is made of a polypropylene resin and has a stiffness [sum of the values for the longitudinal and transverse directions as obtained according to JIS L 1096, 6.19.1, method A (45° cantilever method)] of 70 to 120 mm and a coefficient of static friction of 0.1 to 0.4.

## Description

### Technical Field

The present invention relates to a nonwoven polyolefin resin fabric and an absorbent article used this. In particular, the present invention relates to a nonwoven fabric which has good flexibility, feeling and touch to the skin, and excellent strength, bending rigidity and post-processability, and is capable of using suitably as a material for absorbent article such as disposable diaper and the like.

### Background of the Invention

A nonwoven thermoplastic resin long fiber fabric has been used in many fields, since it has characteristics such as mechanical property like tensile strength, bending rigidity, gas permeability and the like, and at the same time it has excellent continuous spinability and productivity. The thermoplastic resins used to these nonwoven long fiber fabric have been used polyamide resins and polyester resins from melt spinability, fiber characteristic and the like, however polyolefin resins such as polypropylene, polyethylene and the like, which are general purpose resin, have been much used,

Among the nonwoven fabric comprising these polyolefin resins, for example, the polypropylene resin comprises many resins having different degree of crystallinity, even though in homopolymers of propylene, and also it is known that there are many resins having different crystallinity, and also resins having various characteristics such as melting point, strength, elastic modulus and the like by means of copolymers of propylene with ethylene, butene-1 or the like. The nonwoven fabric used such polypropylene resins has good spinability in the case of resin having high degree of crystallinity, however it has problem of poor flexibility and feeling. And moreover, in the case of polypropylene resins having low degree of crystallinity or low melting point, it has flexibility, however there are problems of slimy feeling, and large friction resistance between fibers, fiber and other metal and the like at the time of spinning, and become very worse in the spinability, or usage feeling is poor in articles contacting to the skin such as the absorbent article.

Further, in the case of resins having approximate 90 mol % of isotactic pentad fraction, there are problem that characteristics such as strength, rigidity or the like, and also spinability are relatively favorable, however in the case of used to absorbent articles such as disposable diaper, sanitary napkin, incontinent pad and the like, the obtained nonwoven fabric has not always sufficient using feel such as feeling, touch to the skin and the like.

Various improvements with the spunbonded nonwoven fabric composed of the polypropylene resin long fiber have been proposed. For example, (1) Japanese Patent Laid Open No. 13238/1996 disclosed polypropylene resin compositions for fiber containing crystalline polypropylene resins having ratio (Mw/Mn) of mass average molecular weight (Mw) to number average molecular weight (Mn) of 2 to 15, and isotactic pentad fraction of 96 % or more of 100 mass parts, and fatty acid amide compounds of 0.01 to 1 mass parts.

This official gazette intends to improve poor drawing by fuzzing at the drawing time, by adding lubricants to improve drawability and luster in manufacturing of high strength fibers by high draw ratio of 6 times or more, used high crystalline polypropylene resins. That is, this proposal describes only that the resin differ from the range of crystallinity much used in nonwoven polypropylene resin fabrics, and it is not always favorable in flexibility, feeling and touch to the skin, and it is used suitably to industrial material such as nonwoven fabrics, and it is not nonwoven fabrics for the absorbent article, and it relates to fibers for special use which intended improvement of strength and the like.

On one hand, various methods are proposed to improve feeling, touch to the skin, flexibility and like as nonwoven fabrics used to the absorbent article. First of all, melt blown nonwoven fabric which has fiber diameter of several µm has point of advantage that feeling is soft. However, on the other hand, it is actual circumstances that the melt blown nonwoven fabric is almost not used to the absorbent by itself, since problem of characteristic such as that the nonwoven fabric is low in strength, very low in bending rigidity, fuzzing is occur, threads are easily drawn, incidental looping is easily occurred at the spinning time, rough feeling exist and this stimulate the skin and the like, or productivity is low, thus cost become higher.

On account of this, various laminated nonwoven fabric which are composite feeling of the melt blown nonwoven fabric and respective characteristic such as strength, bending rigidity, productivity and the like of the spunbonded nonwoven fabric are proposed. For example, (2) Japanese Patent Laid Open No. 88056/1990 proposed laminated nonwoven fabrics which specified laminating condition, and (3) Japanese Patent Laid Open No. 143853/1997 proposed a laminated nonwoven fabrics which comprises a composite spunbonded nonwoven fabric composed of two kind of resins having difference of melting points of 10 °C or more and superposed thereon two kind of melt-blown nonwoven fabric having difference of melting points of 10 °C or more, and fused a resin with a nonwoven fabric having low melting point. However, the feeling in this case is carried by the melt-blown nonwoven fabric, the above described problem which is characteristic of the surface of the melt-blown nonwoven fabric is not still dissolved. And also, the composite nonwoven fabric makes complex the manufacturing apparatus, and melting points between the composite resins and fluidity are different, thus spinning is difficult in some case.

On account of this, methods to improve feeling and the like of the spunbonded nonwoven polypropylene fabric having excellent strength and productivity are requested. As the improving method of feeling of the spunbonded nonwoven fabric, first of all to make fine the fiber diameter is considered. However, when the fiber diameter is make too fine, strength is lowered, and also bending rigidity, that is, sturdiness of the nonwoven fabric is decreased, in the manufacturing of the absorbent article such as disposable diaper and the like by post processing, in process of sending out of the nonwoven fabric, sealing and the like, it becomes problem that automation and high speed manufacturing becomes difficult, and large amount and low cost product is not obtained, therefore putting the nonwoven fabric to practical becomes difficult in some case.

On account of this, there is a method using copolymers of propylene with the other olefin. However, also in this case, though feeling is improved considerably, this is due to lowering of bending rigidity (softening of the raw material resin, that is lowering of elastic modulus), lowering of post processability is not changed. And also, spinability is lowered.

Further, (4) Japanese Patent Laid Open No.88459/1998 proposed a long fiber nonwoven fabric composed of a heat fusible conjugate long fiber which comprises at least one kind of low melting point or low softening point resin selected from olefinic copolymer and olefinic terpolymer as the first component, and a crystalline thermoplastic resin as the second component, in which a hydrocarbon based lubricant is contained in at least the first component, and content of the above described hydrocarbon based lubricant is 2 to 20 mass % as the concentration in the fiber.

That is, this proposal intend to improve lowering of spinability caused by using a low melting point of soft propylene random copolymer as polypropylene resins for improvement of flexibility or touch to the skin of the nonwoven fabric, by adding of the hydrocarbon based lubricant. Consequently, characteristics such as heat resistance, strength, bending rigidity and the like, which are essentially afforded to polypropylene resins, especial polypropylene homopolymer, are lost and also the copolymer further contains relatively large amount of the hydrocarbon based lubricant having low molecular weight and low meting point, for example, lowering of heat sealing property, adhesive property and the like of the nonwoven fabric by bleeding of these lubricants is afraid.

Moreover, it is known that a conjugate nonwoven fabric made by conjugate spinning with core sheath structure or side-by-side structure of two kind of resins having different kind and melting point, as described in the above described Japanese Patent Laid Open No. 88459/1998 as the nonwoven fabric. However, although this conjugate fiber nonwoven fabric is improved heat fusibility, in the case of increasing proportion of a comonomer in the low melting point of polypropylene resin, there is slimy feeling of the surface as the nonwoven fabric, dry feeling as the surface material of absorbent article such as disposable diaper, napkin or the like is lowered, and give discomfort feeling in some case.

(5) Japanese Patent Laid Open No. 290381/1999 disclosed a back sheet for the absorptive article which comprises a laminate layered alternately layers composed of polypropylene melt-blown nonwoven fabric and layers composed of polypropylene wet process nonwoven fabric in which one side surface layer of the laminate comprises the polypropylene wet process nonwoven fabric layer. That is, by adopting a short fiber wet process nonwoven fabric, it is intended to secure slipping property of the surface and liquid barrier property. However, the diameter of the fiber of the wet process nonwoven fabric is relatively finer, bending rigidity is lowered, there is a problem of causing complex in the manufacturing method and also lowering the post processability.

Further, (6) Japanese Patent Laid Open No. 293554/1999 disclosed a heat fused nonwoven fabric which comprises conjugate short fibers having fiber diameter of 10 to 15 µm on at least surface layer, in which a coefficient of friction (MIU) of the nonwoven fabric is 0.25 or less, and reflectance per unit area is 1.2 % or more. However, only a nonwoven fabric composed of conjugate short fibers having a fiber diameter of 13 µm and a fiber length of 45 mm, which having a core sheath structure of high density polyethylene as the sheath component and polyester as the core component is disclosed concretely in examples.

Furthermore, as improvement of feeling in a spunbonded nonwoven fabric, (7) Japanese Patent Laid Open No. 92856/1996 disclosed a manufacturing method of a nonwoven fabric having excellent flexibility and touch to the skin, in which a propylene ethylene block copolymer having ethylene content of 0.5 to 8 mass % was used, filament groups having knot on the surface were obtained, thereafter corona treated and thermocompression bonded with heating rolls. However, this method has problem in the production cost and the like, and also has great restriction, which cannot applicate to most general polypropylene homopolymer.

Still more, (8) Japanese Patent Laid Open No.160463/2000 disclosed a flexible nonwoven fabric containing filers composed of polyolefin thermoplastic resin containing olefinic elastomer. And also at the same time, a fabric and a multi layer nonwoven fabric composed of a side-by-side type fiber used the above-described resin and a core sheath type conjugate fiber used the above described resin as core is disclosed. However, the polyolefin thermoplastic resin composing of the fiber of the nonwoven fiber is an object by commonplace way of thinking so as to improve flexibility of the nonwoven fabric, by combining the olefinic elastomer as the soft component, as the result the fiber itself become flexible, sturdiness as the nonwoven fabric is greatly decreased, and post processability is lowered.

As described above, the conventional improving technique of flexibility, feeling, touch to the skin and the like of the spunbonded nonwoven polyolefin resin fabric is not held characteristic of the spunbonded nonwoven polypropylene fabric itself. That is, the technique abandon characteristic of the spunbonded nonwoven fabric which it can be manufactured with good productivity and low cost, and has excellent strength, bending rigidity and post processability, it is owing to only making the fiber diameter into fine, adopting of the soft resin, making short fiber, conjugate spinning, or laminating of these with the spunbonded nonwoven fabric and the like.

Consequently, especially, from post processability such as spinability, bending rigidity or the like, as to the nonwoven fabric, it is actual circumstance that the spunbonded nonwoven polypropylene fabric is used still in spite of poor feeling, touch to the skin or the like.

On account of this, it is strongly excepted improvement of the problem such as flexibility, feeling, touch to the skin and the like, while maintaining strength, gas permeability, especially bending rigidity of the polypropylene spunbonded nonwoven fabric from producer and user of the absorbent article such as disposable diaper.

An object of the present invention is to provide a nonwoven polyolefin fabric and an absorbent article which capable of using suitably for the absorbent article especially such as the disposable diaper, the sanitary napkin and the like, while holding substantially characteristic such as heat resistance, strength, bending rigidity, or the like, which the spunbonded nonwoven polypropylene resin fabric has substantially, and also having excellent flexibility, feeling, touch to the skin or the like.

### Disclosure of the Invention

The present inventors investigate wholeheartedly as to the polyolefin resin, especially the spunbonded nonwoven polypropylene fabric while making the most of function such as gas permeability, flexibility, strength, bending rigidity, heat resistance, post processability, automation suitability and the like afforded to the spunbonded nonwoven polypropylene resin fabric, and also feeling of use, which requested to the end product of the nonwoven fabric, especially the absorbent article, such as spinability, feeling, touch to the skin and the like of the obtained nonwoven fabric. As the result, it is found that feeling of use such as flexibility, feeling, touch to the skin and the like can greatly improved, even though in the spunbonded nonwoven fabric composed of the same fiber diameter, the same area weight and the same resin, by controlling its friction characteristic, and the present invention is completed based on this knowledge.

That is, the present invention provides
(1) A spunbonded nonwoven fabric which comprises a spunbonded nonwoven fabric composed of a polyolefin resin and has an average fiber diameter of 5 to 60 µm, area weight of 5 to 200 g/m² and a coefficient of static friction of 0.1 to 0.4.
(2) The spunbonded nonwoven fabric according to the above described (1), wherein the nonwoven fabric contains a lubricant.
(3) The spunbonded nonwoven fabric according to the above described (1) or (2), wherein the lubricant is a fatty acid amide compound, and its content is 0.05 to 1.0 mass %.
(4) The spunbonded nonwoven fabric according to any one of the above described (1) to (3), wherein the polyolefin resin is polypropylene resin.
(5) The spunbonded nonwoven fabric according to any one of the above described (1) to (4), wherein the fiber is hydrophilicity imparting treated.
And also, the present invention provides
(6) A spunbonded nonwoven fabric which comprises a spunbonded nonwoven polypropylene resin fabric having stiffness(bending resistance), which is the sum of the values for the longitudinal and transverse directions as obtained according to JIS L1096 6.19.1, method A that is 45° cantilever method, of 70 to 120 mm, and a coefficient of static friction of 0.1 to 0.4.
(7) The spunbonded nonwoven fabric according to the above described (1), wherein the nonwoven fabric has the average fiber diameter of 10 to 30 µm, and area weight of 10 to 30 g/m².
(8) The spunbonded nonwoven fabric according to the above described (7) or (8), wherein the nonwoven fabric contains the lubricant of 0.15 to 1.0 mass %.
(9) The spunbonded nonwoven fabric according to the above described (8), wherein the lubricant is a fatty acid amide compound.
(10) The spunbonded nonwoven fabric according to any one of the above described (6) to (9), wherein the fiber constituting of the nonwoven fabric is hydrophilicity imparting treated.
Further, the present invention provides
(11) An absorbent article made by using the nonwoven fabric according to any one of the above described (1) to (10).
(12) The absorbent article according to the above described (11), wherein the absorbent article is a disposable diaper, a sanitary napkin or incontinence pad.

### Brief Description of Drawing

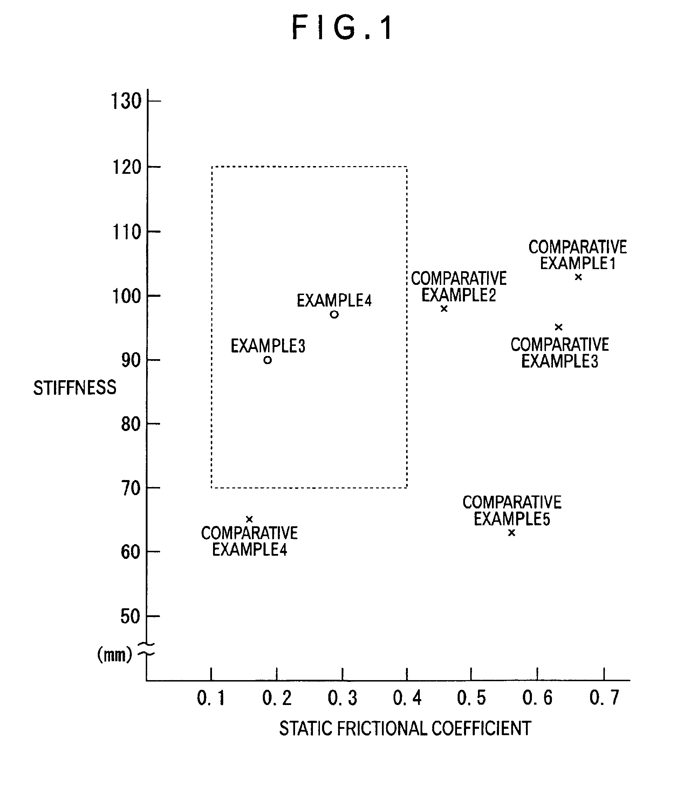

Figure 1 is an illustration view of relation of degree of bending rigidity and coefficient of static friction of the spunbonded nonwoven fabric in examples of the present invention and comparative example.

### Best Mode to Carrying the Invention

In the following description, the present invention will be described in further detail.

### [The first embodiment]

The nonwoven polyolefin resin fabric of the present invention comprises the spunbonded nonwoven fabric having the average fiber diameter of 5 to 60 µm,area weight of 5 to 200 g/m², and the coefficient of static friction of 0.1 to 0.4. The nonwoven fabric of the present invention may be any object satisfying the range of the coefficient of static friction of 0.1 to 0.4 as the nonwoven fabric, its means to control the coefficient of static friction may be optional, and is considered adoption of various means. However, the reason why the spunbonded nonwoven fabric of the present invention has excellent flexibility and feeling is considered an effect by improvement of slipping property among long fibers, so that it is preferred a material which improves slipping property of not only fibers on the surface part of the nonwoven fabric, but also fibers as whole the nonwoven fabric, as the result, and a coefficient of static friction as the surface characteristic become in a specific range.

### (Basic characteristic of Nonwoven fabric)

The fiber diameter of the fiber composing the spunbonded nonwoven fabric is 5 to 60 µm, preferably 5 to 40 µm, and 15 to 30 µm for especially the absorptive article such as the disposable diaper or the like. Here, in the case of 5 µm or less, strength and bending rigidity are insufficient, and also in the some case post processability is lowered, and at the same time the using field is greatly limited. Moreover, in the case of 60 µm or more, flexibility is lowered, characteristics as the nonwoven fabric is lost, and also in the some case especially application to the absorptive article, various clothes becomes difficult.

Moreover, area weight of the spunbonded nonwoven fabric of the present invention is in the range of 5 to 200 g/m², preferably 7 to 60 g/m², especially 10 to 30 g/m² for the absorbent article such as the disposable diaper or the like. Here, in the case of area weight of 5 g/m² or less, in some case strength is insufficient, bending rigidity (sturdiness) is lower, and post processability is fallen. Moreover, in the case of 200 g/m² or more, flexibility, gas permeability or the like is lowered, thus characteristics as the nonwoven fabric are lost, and in some case usage becomes difficult due to use.

The spunbonded nonwoven fabric can select/combine properly the resin, the fiber diameter, area weight and the like, based on the use of the nonwoven fabric, required property, gas permeability, non-water permeability or the like.

The spunbonded nonwoven polyolefin resin fabric of the present invention is not especially restricted as far as it is the spunbonded nonwoven fabric, and it can produce by various manufacturing process. Moreover, the bonding form can adopt embossing, calendaring, heat adhesion such as hot air or the like, mechanical entangling such as needle punching, water punching and the like. However, heat adhesion by embossing is preferred from productivity.

### (Coefficient of Static Friction)

The characteristic of the present invention is that the nonwoven fabric of the spunbonded nonwoven fabric comprising conventional well known polyolefin resin has the coefficient of static friction of 0.1 to 0.4, preferably 0.12 to 0.35. Here, in the case of the coefficient of static friction is 0.1 or less, processability is lowered by over slippage in the post processability, and at the same time the usage amount of additives or surface treatment agent adopted general is necessary due to lowering of the coefficient of static friction, in some case economic performance is fallen. Further, in some case thermal sealing at the time of post processing, conjugation property by adhesives and the like is trended toward poor, thus such case is not preferred. Moreover, in the case of 0.4 or more, improving effect of feel of usage such as flexibility, feeling, touch to the skin become insufficient.

### (Measurement of Coefficient of Static Friction)

The coefficient of the spunbonded nonwoven fabric of the present invention can determine according to ASTM-D 1894. Concretely, based on measuring conditions of:
Measuring machine of a coefficient of static friction: made of Toyo Seiki Seisakusho Co. Ltd., AN type
Loading plate : 63.6mm× 102.2m × 19.4mn (height)
Load: 8.87N of iron plate
Incline speed: 2.7 degree/second
sliding angle θ was determined by piling up measuring surfaces of the nonwoven fabric, tan θ was obtained, and made it the coefficient of static friction. The smaller the numerical value the better the slipping property.

### (Polypropylene Resin)

The polyolefin resin used in the spunbonded nonwoven polyolefin resin fabric of the present invention is not especially restricted, it can include a homopolymer of propylene, copolymer of propylene with at least one of α-olefin such as ethylene, butene-1, 4-methyl-pentene-1, hexane-1, octen-1 or the like. These polypropylene resins are selected properly from those that having difference of various crystallinity, molecular weight and distribution of molecular weight from selection of catalysts at the time of polymerization, polymerization condition or the like, based on the property required in the nonwoven fabric. This selection is examined from the points of strength, bending rigidity, use and the like, but from spinability, vending rigidity, slimy feeling or the like as described above, using of the homopolymer of propylene or the polypropylene copolymer with low ratio of copolymerization is preferred.

Here, crystallinity is selected from a range from 88 to 95 mol %, preferably 89 to 93 mol % in the isotactic pentad fraction in the case of using the nonwoven fabric as the disposable diaper and the like. Here, the isotactic pentad fraction (IPF) is an isotactic fraction in the pentad unit in the polypropylene molecular chain, which is measured by using nuclear magnetic resonance spectrum (¹³C-NMR) by an isotope carbon for example described in Macromolecules. vol. 28, No16, pp5403 (1995).

Moreover, melt flow rate (MFR) [according to JIS K 7210, measuring temperature : 230 °C, measuring load : 21.18N] of the polypropylene resin is in the range of 5 to 200 g/10 min, preferably 10 to 100 g/10 min. In particular, those that in the range of 30 to 80 g/10 min is suitable for the absorptive material.

### (Polyethylene Resin)

Next, polyethylene resins include a homopolymer of ethylene, copolymers of ethylene with α-olefin having 3 to 10 carbon atoms such as propylene, butene-1, 4-methyl-pentene-1, hexane-1, octane-1 or the like, and copolymers of ethylene with a polymerizable monomer such as vinyl acetate, acrylic acid or the like. In particular, the above described ethylene- α -olefin copolymer having density of 880 to 960 kg/m³, preferably 900 to 950 kg/ m³,melting point in the range of 100 to 140 °C, preferably 110 to 130 °C, and melt flow rate (MFR) [according to JIS K 7210, measuring temperature : 190 °C, measuring load : 21.18N] of 5 to 60 g/10 min, preferably 10 to 50 g/10 min is used preferably from points of spinability, melting point, bending rigidity.

Moreover, these polypropylene resins and polyethylene resins may be mixtures of two kind or more respectively, and can use as resin compositions containing other ethylene resin, propylene resin, thermoplastic elastomer or the like of less than 50 mass %.

### (Controlling Method of Coefficient of Static Friction)

Next, means to obtain the spunbonded nonwoven fabric having the coefficient of static friction in the range of 0.1 to 0.4 are not restricted especially, and various means are included. Concretely, it can exemplify roughly divided to (1) melt spinning process by combining a lubricant into a polyolefin resin for spinning, and (2) surface treating process to the fiber after spinning.

### (Lubricant)

Here, the lubricant is not especially restricted, and include fatty acid amide compound, fatty acid compound, paraffin and hydrocarbon resin, silicone compound, silicone polymer, fluorine compound, fluorine polymer such as copolymer of tetrafluoroethylene with propylene, copolymer of vinylidene fluoride with hexafluoroethylene or the like, or these mixture. Among them the fatty acid amide compound is preferably used.

The fatty acid amide compound include fatty acid monoamide compound, fatty acid diamide compound, saturated fatty acid monoamide compound, unsaturated fatty acid diamide compound. Concretely, it includes amide laurate, amide myristate, amide palmitate, amide stearate, amide behenate, amide oleate, amide erucate, amide montanate, amide N,N'-methylene-bis-laurate, amide N,N'-methylene-bis-myristate, amide N,N'-methylene-bis-palmitate, amide N,N'-methylene-bis-behenate, amide N,N'-methylene-bis-oleate, amide N,N'-methylene-bis-erucate, amide N,N'-ethylene-bis-oleate, amide N,N'-ethylene-bis-erucate and the like, and also these can be used by combining plural kind of substances.

Among these fatty acid amide compounds, amide erucate, which is an unsaturated fatty acid monoamide compound, is preferably used. The reason why it is used is they are suitable to decrease the coefficient of static friction of the nonwoven fabric, by decreasing of spinability by exposing unnecessarily the fatty acid amide on the surface at the time of spinning of the fiber of the nonwoven fabric, and ageing of the nonwoven fabric containing the fatty acid amide compound described below. Content of the fatty acid amide compound in the polyolefin resin is in the range of 0.05 to 1 mass %, preferably 0.1 to 0.5 mass %. This content is decided by judging synthetically kind of the polyolefin resin, the resin characteristics such as crystallinity, MFR and the like, kind of the fatty acid amide compound, required property of the obtained nonwoven fabric, ageing condition and the like.

Consequently, for example, in the case of using amide erucate in the homopolymer of propylene having isotactic pentad fraction of around 90 mol %, it is preferred in the range of 0.1 to 0.5 mass %, especially 0.2 to 0.4 mass %. In this case, although it depends on ageing treatment condition, if it is 0.2 mass % or less, in some cases it is difficult to control the coefficient of static friction of the nonwoven fabric in the range of 0.1 to 0.4, and if it is 0.4 mass % or more, in some cases the amount of the amide erucate on the surface of the nonwoven fabric is much, it becomes cause of worse of appearance such as generation of white powder or the like, or lowering of heat fusion property and post processability.

Further, the spunbonded nonwoven polyolefin resin fabric of the present invention can be added well-known additives component used generally in the nonwoven fabric for use or imparting characteristic or the like. These well known additives components include neutralizating agents such as calcium stearate, hydrotalcite or the like, antioxidants such as phenol based, phosphorus based, sulfur based or the like, heat stabilizers, nucleus forming agents, UV absorbers, light stabilizers, antistatic agents, flame retardants, pigments, dyes, or inorganic powders such as silica, talc, calcium carbonate, calcium oxide, magnesium oxide or the like.

### (Melt Spinning)

The spunbonded nonwoven polyolefin resin fabric of the present invention make a spunbonded primary nonwoven fabric by melt spinning a mixture which is dry blended fixed amount of lubricant such as fatty acid amide or the like and additives component added as the need arises to a polyolefin resin.

Here, the spunbonded nonwoven fabric can be obtained, for example, a primary nonwoven fabric by well known method which comprises melt extruding the raw material polyolefin resin having the above described combination, spinning it from a spinneret for spinning, taking up the spun fiber with an airborne tracking apparatus such as air sucker or the like, opening as the need arises, collecting fibers with air flow by a web collecting apparatus such as a net conveyer or the like, partial fusing with heating means such as heated air, heating roll or the like, thereafter winding up.

Further, this spunbonded nonwoven polyolefin resin fabric is a nonwoven fabric comprising ordinarily polyolefin resin alone, but it may be a conjugate fiber nonwoven fabric comprising a polyolefin resin of at least 50 % on the surface of the fiber.

These composite fiber nonwoven fabric may be used the composite fiber having core-sheath structure composing of a polyolefin resin as the sheath component and a resin having higher melting point than the sheath component resin excepting the polyolefin resin such as a polyamide resin, polyester resin or the like as the core component, or the conjugate fiber having side-by-side structure which comprises the polyolefin resin of ordinarily 50 mass % or more of fibers and the other resin in the rest. Further, this core-sheath structure conjugate fiber and side-by-side structure conjugate fiber may be, of course, combination of two kind of different polyolefin resins among polyolefin resins.

### (Ageing Processing)

Thus obtained spunbonded primary nonwoven fabric is excellent in spinability, however in some cases the coefficient of static friction specified by the nonwoven fabric of the present invention is not expressed by itself, by the kind of the lubricant, especially in the case of the fatty acid amide compound. In this case, to make the spunbonded nonwoven polyolefin resin fabric of the present invention, by only after ageing this primary nonwoven fabric under heating, it can be the range of the coefficient of static friction which is specified by the present invention. In the conventional nonwoven fabric manufacturing apparatus, such ageing apparatus is not included, generally ageing was not performed

Here, ageing condition is differed due to kind of the polyolefin resin, resin characteristic such as degree of crystallinity, density, melting point or the like, kind and melting point of contained the fatty acid amide compound and solubility to the polyolefin resin or the like. Consequently, by considering characteristic of the polyolefin resin which is the raw material of the nonwoven fabric, or characteristic of the fatty acid amide compound as the lubricant, and by considering the range of the coefficient of static friction in the range of 0.1 to 0.4, required characteristic such as flexibility, feeling, touch to the skin or the like required to the end product, concretely the treating condition is decided experimentally.

For example, the spunbonded nonwoven polyolefin resin fabric of the present invention is obtained by ageing a spunbonded nonwoven fabric made by melt spinning the polyolefin resin which containing the fatty acid amide compound at a temperature of 30 to 60 °C for around 1 to 50 hours. For example, in the homopolymer of polypropylene, as a concrete example in the case of that having isotactic pentad fraction of around 90 mol % and content of amide erucate of 0.3 mass %, the following ageing treatment condition can set up.

In the case of the ageing temperature of 40 °C, the ageing time is 5 to 50 hours, preferably about 8 to 12 hours. Moreover, in the case of the ageing time is 24 hours, the ageing temperature is 32 to 50 °C, preferably about 33 to 40 °C. When the ageing condition is milder than the range described above, lowering of the coefficient of static friction requires longer time, in some cases productivity is lowered. Further, when the ageing condition is severer than the above described range, in some cases the coefficient of static friction become higher reversely, thus it is not preferred.

This ageing treatment can ordinarily carried out in an ageing room which heated air is circulated, by arranging core pipes in the state of winding the nonwoven fabric in roll form. When this ageing is performed, even though the nonwoven fabric is wound state in roll form, the nonwoven fabric is obtained approximate uniform effect of ageing treatment due to gas permeability of the nonwoven fabric. Further, ageing can also carried out by roll heating and/or heated air, the nonwoven fabric is not wound state, but while it is running between rolls.

### (Surface Treatment)

Next, the other method to give the coefficient of static friction of the nonwoven fabric of the present invention is a method by surface treatment of the fiber of the nonwoven fabric obtained by spinning. As the surface treating agent, for example, a pollution of dimethyl siloxane, methyl hydrogen polysiloxane, compound containing fatty acid amide can be used. However, this surface treatment has problem that cannot treat into inner pert in some cases, due to wet treating process, drying process and thickness of the nonwoven fabric. Therefore, in many cases the methods by melt blending of the lubricant described above is preferred, due to form, use, range of the coefficient of static friction or the like of the nonwoven fabric.

The spunbonded nonwoven fabric comprising the polyolefin resin of the present is generally hydrophobicity, owing to use of the absorptive articles and the like, for example, in the case of using as the top material of the disposable diaper or sanitary napkin or the like, in some cases hydrophobicity of at least water or the like permeable is required. In this case the nonwoven fabric can do hydrophobicity imparting treatment.

This hydrophobicity imparting treatment include introduction of hydrophilic groups such as carboxyl group or the like by ozone treatment, or surface treatment by hydrophilic compounds, however treatment by solution of hydrophilic compounds is preferred in the point of the effect. As the treating method, spraying method, coating method, immersing method and the like can be exemplified. Moreover, the hydrophilic compound can be exemplified, for example, alkyl-ester such as polyalcohol having 8 to 26 carbon atoms containing polyoxyethylene, alkyl-ether, polyether containing fatty acid amide group, fatty acid monoglyceride, sorbitol ester derivative, alkyl phosphate metal salt, polyoxyethylene alkyl ether sulfate metal salt, alkyl sulfosuccinate metal salt, sugar derivative having glucose ring and the like.

The spunbonded nonwoven fabric composed of the polyolefin resin of the present invention is maintained essential characteristic of the nonwoven fabric which has the polyolefin resin itself, by lowering of the coefficient of static friction by the ageing treatment or the surface treatment, and flexibility, feeling, touching feel such as touch to the skin, and usage feel are improved markedly. Consequently, it can be used as materials for various absorbent article (such as sanitary material and the like), various clothing materials, material for medical treatment, material for wrapping and the like.

### (Forming of Multilayer)

The spunbonded nonwoven fabric can make a multilayer material with other nonwoven fabric, thermoplastic resin film, paper or the like. However, in the case of used as multilayer object, at least one side of the nonwoven fabric is used the nonwoven fabric of the present invention which satisfy the above described characteristics such as the coefficient of static friction of 0.1 to 0.4. And, the other nonwoven fabric in the case of using the spunbonded nonwoven polyolefin resin fabric in the multilayer nonwoven fabric may be a nonwoven fabric which composed of not the polyolefin resin but the polyamide resin or the polyester resin.

The characteristic related to feel of the nonwoven fabric is the problem on the surface of the nonwoven fabric material, to improve the characteristics such as strength, moisture permeability, gas permeability, powder barrier property, heat fusibility and the like, it can also be made the multilayer material with general polyolefin resin nonwoven fabric containing no the fatty acid amide compound, other thermoplastic resin nonwoven fabric, the moisture permeable film, water resistant film, water-proofing film or the like.

In this case, usually the spunbonded nonwoven polyolefin resin fabric after the above described ageing treatment is laminated so as to exposure on at least one side. However, owing to the case, it can also be ageing treated after lamination with other nonwoven fabric or film or the like. The other nonwoven fabric in this case include the melt-blown nonwoven fabric, short fiber nonwoven fabric or the like. For example, a laminating method where the melt-blown nonwoven fabric is spun on the spunbonded nonwoven fabric, and further spunbonded nonwoven fabric is manufactured in multi-step and continuously thereon, a multi-layer nonwoven fabric is previously manufactured by containing fatty acid amide into a polyolefin resin for at least one side of spunbonded nonwoven fabric, and then this multi-layer nonwoven fabric is ageing treated is given.

Here, the nonwoven fabric except the polyolefin resin can include a nonwoven fabric, for example, of the polyester resin, the polyamide resin, especially having melting point of 150 °C or more, particularly 150 to 300 °C. Here, the polyester resin can include homopolyester such as polyethylene terephthalate, polybutylene terephthalate, polytrimethylene terephthalate, polynaphthalene terephthalate and the like, and copolyester which copolymerized with other component, in which the homopolyester is main component unit, and further these blended polyester.

The polyamide resin can exemplify nylon 6 (polycaprolactamide), nylon 6,6 (polyhexamethylene adipamide), nylon 6,10 (polyhexamethylene sebacamide), nylon 11 (polyundecane amide), nylon 7 (poly-ω-aminoheptanic acid), nylon 9 (poly-ω-amino-nonaic acid), nylon 12 (polylauric amide) and the like. Among them, nylon 6 and nylon 6,6 are used preferably.

As the laminating means to form this multilayer nonwoven fabric, various laminating means such as heat adhesion, adhesion with adhesives and the like is given. However, simple and low cost laminate heat adhesion means, especially heat embossing roll method can be adopted. This embossing roll method can laminate by using well-known laminating apparatus with an embossing roll and a flat roll. Here, the embossing roll can adopt various shape embossing pattern, there are lattice state in which each fused part is continued, independent lattice state, optional distribution and the like. Moreover, emboss area fraction is in the range of approximate 5 to 40 %.

Further, the spunbonded nonwoven polyolefin resin fabric can also make the mutilayer material with moisture permeable resin layer (film), the water resistant resin layer (film), and the water-proofing resin layer (film) in addition to lamination with the other nonwoven fabric. In this case, an extruding laminating method, a heat embossing roll method, a dry laminating method and the like can adopt.

The heat embossing roll lamination condition is different by melting point of the spunbonded nonwoven fabric or the other nonwoven fabric, and making either layer of other film the emboss side, the condition is selected properly by taking respective factor into consideration. These emboss pattern, emboss area fraction, temperature, pressure and the like can be properly selected depending on the fiber diameter, thickness, area weight, gas permeability, processing speed of each nonwoven fabric, and further melting point, thickness and the like of other nonwoven fabric film or the like.

The spunbonded nonwoven fabric is used for, including the disposable diaper, sanitary napkin or incontinence pad, medical treatment, clothing and wrapping.

### [Examples based on the First Embodiment]

In the following, the present invention will be illustrated in further detail based on the manufacturing examples of the spunbonded nonwoven fabric based on the first embodiment. However, the present invention is not limited by these examples.

### (Example 1)

To a crystalline polypropylene resin [isotactic pentad fraction : 91 mol %, MFR : 60 g/10 min, melting point:160 °C] 100 mass parts, a phenol based antioxidant (made of Ciba Special Chemical Company, Irganox 1010):0.035 mass parts, phosphorus based antioxidant (made of Sando Company, Sandostab P-EPQ):0.035 mass parts, a neutralizer (made of Kyodo Yakuhin Co. Ltd., calcium stearate) 0.025 mass parts and amide erucate: 0.3 mass parts were dry blended with a super-mixer, thereafter melt kneading at 220 °C by using 65 mm φ extruder, and melt spun by extruding from the spinneret. The spinneret in this case was diameter of hole of the spinneret: 0.3 mm, and numbers of it in width direction : 200, and extrusion direction : 15.

Then, the spun fiber group were introduced into an air sucker and tracked and drawn, and collected on a belt having a suction apparatus, and then it was sent to heated embossing rolls [ an embossing roll at 140 °C/a flat roll at 140 °C ] and partly adhered, and then rolled it onto a paper tube, thus a primary nonwoven fabric was obtained. The obtained rolled nonwoven fabric was ageing treated in an ageing condition of 40 °C • 24 hours to obtain a spunbonded nonwoven polyolefin resin fabric of the present invention. The obtained nonwoven fabric had an average fiber diameter : 16 µm, area weight: 20 g/m², a coefficient of static friction : 0.19, degree of bending rigidity : 5.3 cm, touch to the skin : ⓞ, feeling : ⓞ. and end breakage or jogging in the spinning process was not observed, and it can spun stably.

Further, evaluation of the nonwoven fabric was performed based on the following description.

### (1) Coefficient of Static Friction

Measurement was performed according to the measuring method of the coefficient of static friction of ASTM-D 1894. And, the detail was described above.

### (2) Degree of Bending Rigidity [Longitudinal Direction] (cm)

Measurement was performed according to JIS L 1096 (45° cantilever method).

### (3) Touch to the skin • feeling

A functional test by touch to the skin • handle by 20 monitors was performed, and evaluation of ⓞ,○ and Δ was performed.

### (Example 2)

An experiment was performed according to example 1 except that changing the content of amide erucate to 0.4 mass parts, the aperture of spinneret to 0.3 mm, extruding speed and belt speed. The obtained nonwoven fabric had an average fiber diameter : 24 µm, area weight: 20 g/m², a coefficient of static friction : 0.19, of stiffness : 5.3 cm, touch to the skin : ⓞ, feeling : ⓞ. And end breakage or jogging in the spinning process was not observed, and it can spun stably.

### [The Second Embodiment]

A spunbonded nonwoven polypropylene resin fabric of the present invention is a spunbonded nonwoven fabric which has stiffhess [sum of the value for the longitudinal and transverse directions as obtained according to JIS L 1096, 6.19 1, method A (45° cantilever method )] of 70 to 120 mm and a coefficient of static friction of 0.1 to 0.4.

### (Stiffness)

That is, spunbonded nonwoven fabric has, first of all, has stiffness [sum of the values for the longitudinal and transverse directions as obtained according to JIS L 1096, 6.19.1, method A (45° cantilever method)] of 70 to 120 mm. As the item of evaluation of performance of the spunbonded nonwoven fabric is considered feeling together with strength and the like as important. This feeling is considered as combination of softness, hardness, resiliency, restitution property, feeling of cold temperature, luster, draping property and the like, and it is well known that feeling is different greatly by the manufacturing method, especially adhesion method.

Among these properties, degree of bending rigidity by the evaluation test of flexibility, which is index of softness, is standardized various test method depending to characteristic of the nonwoven fabric. The spunbonded nonwoven fabric of the present invention is specified by the measuring value by the above described 45° cantilever method, which is suited to evaluation of flexibility of the most general nonwoven fabric. The stiffness of the spunbonded nonwoven fabric is usually observed difference of stiffness, in flow direction (longitudinal) and direction at right to flow (transverse) in the manufacturing process of the nonwoven fabric. Consequently, in the spunbonded nonwoven fabric of the present invention, to make the average this property of direction, stiffness was specified with sum of stiffness in longitudinal • transverse direction. (In the following, sum of values of stiffness far the longitudinal and transverse direction is used as the stiffness as far as especially not making any comment.

The stiffness of the spunbonded nonwoven polypropylene resin fabric, which is general purpose article having the fiber diameter of approximate 20 µm and area weight of 20 g/m², is approximate 70 to 120 mm. It is said that the stiffness is desirable low for feeling. As means to lower stiffness, it has been investigated means which to finer the fiber diameter, to decrease area weight, to adopt low crystalline resin as the polypropylene resin, and further to lower elastic modulus of the polypropylene resin by combining soft resin or thermoplastic elastomer to the polypropylene resin and the like.

However, the spunbonded nonwoven polypropylene resin fabric require not only feeling but also balance of synthetic properties. The method to improve feeling by lowering the degree of stiffness described above is that to soften the polypropylene resin itself, or to decrease fiber diameter or area weight. This can to say improving means that sacrifice strength and moderate flexibility, which are fundamental special character of the spunbonded nonwoven polypropylene resin fabric.

Consequently, at manufacturing time of the spunbonded nonwoven fabric or, especially, in processing into the end product such as disposable diaper, stabilization of the nonwoven fabric at the post processing time such as sending out of the nonwoven fabric, heat sealing and the like is not maintained, from point of stabilization of quality, productivity and the like, there is limit in technique to only lower the stiffness. Consequently, the spunbonded nonwoven polypropylene resin fabric, which is much consumed, has been used that having stiffness of 70 to 120 mm in spite of existing dissatisfaction in feeling, touch to the skin and the like. And also, for example, even though a spunbonded nonwoven fabric which combined a thermoplastic elastomer of 10 mass % into a polypropylene resin, decreasing of the stiffness is slight, and when combining amount of the thermoplastic elastomer make more much, not only the post processability decrease but also spinability lower.

### (Coefficient of Static Friction)

Moreover, by investigation of the inventors of the present invention, it was found that the numerical value of the stiffness itself do not showed directly feeling of the spunbonded nonwoven polypropylene resin fabric as described above. That is, it was found that feeling which feel on hand or skin of human can not evaluated by the numerical value of the stiffness alone.

The spunbonded nonwoven polypropylene resin of the present invention has characteristic which satisfy in addition to the stiffness of 70 to 120 mm, preferably 75 to 115 mm as the first, and the coefficient of static friction in the range of 0.1 to 0.4, preferably 0.12 to 0.36 as the second. Although the reason why the coefficient of static friction of the spunbonded nonwoven fabric relates greatly to feeling of the nonwoven fabric is not always clear, it is considered that slippage between fibers which constitute the nonwoven fabric become favorable in the case of touching to the hand or skin, and the whole nonwoven fabric become easy deformable. On account of this, evaluation of feeling of the spunbonded nonwoven fabric is difficult by only the stiffness, it became clear that even though the equal stiffness, it was different greatly by the coefficient of static friction.

The coefficient of static friction of the spunbonded nonwoven polypropylene resin fabric of the present invention may be controlled into 0.1 to 0.4, the means is optional, and is concerned adoption of various means. However, it is considered that the reason why the spunbonded nonwoven fabric of the present invention has excellent flexibility and feeling is the effect by improving of slipping property between the above described long fibers, therefore not only slipping property of the fiber of the surface part of the nonwoven fabric, but also slipping property of the fiber as the whole nonwoven fabric are improved, as the result the coefficient of static friction as the surface characteristic is in the specific range.

The characteristic of the present invention is that a spunbonded nonwoven fabric is in addition to having specific range of stiffness which secure strength, sturdiness, post processability and the like as conventional well-known spunbonded nonwoven polypropylene resin nonwoven fabric, having the above described coefficient of static friction of 0.1 to 0.4, preferably 0.12 to 0.36. Here, when the coefficient of static friction is 0.1 or less, in addition to lower processability reversely by over-slippage in the post processing, owing to lowering the coefficient of static friction, it require more much usage amount of additives or surface treating agent adopted generally, thus it becomes worse economically in some case Further, there is trend to lower conjugativity by heat sealing, adhesives and the like at the time of post processing, and it is not preferred in some case. And, when the coefficient of static friction is 0.4 or more, the improving effect of usage feel such as flexibility, feeling, touch to the skin becomes insufficient.

Moreover, measurement of the coefficient of static friction is as described in paragraph of "measurement of a coefficient of static friction" in the first embodiment described above.

### (Basic Characteristic of Nonwoven Fabric)

The spunbonded nonwoven polypropylene resin fabric has preferably an average fiber diameter of 10 to 30 µm and area weight of 10 to 30 g/m², and more preferably the average fiber diameter of 10 to 25 µm and area weight of 15 to 25 g/m². Here, when the average fiber diameter is 10 µm or less and area weight is 10 g/m² or less, securing the range of strength and stiffness is difficult, as the result post processability become lower. And, when the average fiber diameter is 30 µm or more and area weight is 30 g/m², securing of stiffhess and feeling is difficult in some case. And flexibility become lower, characteristics as the nonwoven fabric is decreased, especially application to the absorbent article and the like is difficult in some case, and its using field is restricted greatly.

The spunbonded nonwoven polypropylene resin fabric of the present invention can select/combine properly resin, fiber diameter, area weight and the like based on the use of the nonwoven fabric, required property, gas permeability, non-water permeability and the like.

The nonwoven polyopropylene resin fabric used in the present invention is not especially restricted as far as it is the spunbonded nonwoven fabric, and it can produce by various manufacturing process. Moreover, the bonding form can adopt embossing, calendaring, heat adhesion such as hot air or the like, mechanical entangling such as needle punching, water punching and the like. However, heat adhesion by using heat embossing method is preferred from securing of productivity stiffhess and the like.

This heat embossing roll method is performed by using well-known laminating apparatus having an embossing roll and a flat roll. Here, the embossing roll can adopt various shape embossing pattern, there are lattice state in which each fused part is continued, independent lattice state, optional distribution and the like. And, emboss area fraction is in the range of approximate 5 to 40 %. Here, when emboss area fraction is 5 % or less, it come off the lower limit of the rang of stiffness, and when the emboss area fraction is 40 % or more, it come off the upper limit of stiffness, and at the same time flexibility is easily decreased in some case. And, when embossing pressure is low, usage feel such as flexibility, feeling, touch to the skin is favorable, however reversely stiffness is lowered, and post processability is decreased markedly, thus it is not practical.
Consequently, condition of heat embossing method is selected properly by taking shape of embossing part, pitch between emboss adhering parts, roll temperature and the like, and stiffness into consideration.

### (Polypropylene Resin)

In this embodiment, the spunbonded nonwoven fabric used the polypropylene resin described in previously in the first embodiment is suitable. Since the propylene resin is as described previously, redundant explanation is omitted.

These polypropylene resins may be mixtures of two kind or more, and can use as resin compositions containing other ethylene resin, propylene resin, thermoplastic elastomer or the like of less than 30 mass % according to necessity.

### (Method for Control Coefficient of Static Friction)

In this embodiment, the coefficient of static friction is controlled by applying the lubricant to the fiber of the nonwoven fabric as same as the first embodiment described above. Since the "lubricant", "melt spinning", "ageing" and "surface treatment" is as described previously in explanation of the first embodiment, redundant explanation is omitted here.

### (Formation of Multilayer)

Moreover, in this embodiment, the nonwoven fabric may be made the multiplayer nonwoven fabric as same as the first embodiment described previously. With regard to formation of multiplayer, since it is as described previously in the paragraph of "formation of multilayer", redundant explanation is omitted here.

### [Examples based on the Second Embodiment]

In the following, the present invention will be illustrated in further detail based on manufacturing examples of the spunbonded nonwoven fabric based on the second embodiment. However, the present invention is not limited by these examples.

### (Example 3 to 4, and Comparative Example 1 to 5)

To a crystalline polypropylene resin [isotactic pentad fraction : 91 mol %, MFR : 60 g/10 min, melting point: 160 °C] 100 mass parts, a phenol based antioxidant (made of Ciba Special Chemical Company, Irganox 1010):0.035 mass parts, phosphorus based antioxidant (made of Sando Company, Sandostab P-EPQ):0.035 mass parts, a neutralizer (made of Kyodo Yakuhin Co. Ltd., calcium stearate) 0.025 mass parts and amide erucate : [mass parts listed in Table 1] were dry blended with a super-mixer, thereafter melt kneading at 220 °C by using 65 mm φ extruder, and melt spun by extruding from the spinneret. The spinneret in this case was diameter of orifice of the spinneret in example 1,2 and comparative example 1 to 4: 0.4 mm, and in comparative example 5 : 0.3 mm, and numbers of it in width direction : 200, and extrusion direction : 15.

Then, the spun fiber group were introduced into an air sucker and tracked and drawn, and collected on a belt having a suction apparatus, and then it was sent to heated embossing rolls [the condition listed in Table 1] and partly adhered, and then rolled it onto a paper tube, thus a primary nonwoven fabric was obtained. By alternating manufacturing condition of the nonwoven fabric, nonwoven fabric having different average fiber diameter, area weight and degree of adhesion were obtained.

The obtained rolled nonwoven fabric was ageing treated in an ageing condition of 40 °C • 24 hours to obtain a spunbonded nonwoven polypropylene resin fabric. The average fiber diameter, area weight, the coefficient of static friction, stiffness, feeling, post processability and result of spinability of the obtained nonwoven fabric are listed in Table 2.

Further, evaluation of the nonwoven fabric was performed based on the following description.

### (1) Coefficient of Static Friction

Measurement was performed according to the measuring method of the coefficient of static friction of ASTM-D 1894. And, the detail was described above.

### (2) Stiffness [Sun of the Values for the Longitudinal and Transverse Directions]

Measurement was performed according to JIS L 1096 6.19.1, method A (45° cantilever method).

### (3) Feeling

A functional test by touch to the skin • handle by 20 monitors was performed, and evaluation of ⓞ,○ and Δ was performed.

### (4) Post Processability

To the spunbonded nonwoven fabric, by using a nozzle discharge system fiber spray die, an ethylene-vinyl acetate based hotmelt adhesive H-6805 [made of Nitta Findorei Co. Ltd.,] was sprayed so as to be coating amount of 4 g/m² in fibrous state. And then, it was superposed thereon a drawn PE film containing inorganic filler "Poram PU35" (thickness 35 µm) made of Tokuyama and laminated together to obtained a nonwoven fabric laminate. When this lamination, those that got wrinkle • meander • neck-in made Δ, and that laminated without trouble made ⓞ.

**(Table 1)**

| | amide erucate | ageing | embossing condition | | | |
|---|---|---|---|---|---|---|
| | mass parts | do or don't | area fraction | pressure | temperature (°C) | spinality |
| | | | % | N / cm | emboss side emboss side /flat side | |
| example3 | 0.2 | do | 20 | 500 | 135/135 | ○ |
| example4 | 0.2 | do | 20 | 500 | 135/135 | ○ |
| comparative example 1 | No | don't | 20 | 500 | 135/135 | ○ |
| comparative example2 | 0.1 | do | 20 | 500 | 135/135 | ○ |
| comparative example3 | No | don't | 20 | 500 | 135/135 | ○ |
| comparative example4 | 0.4 | do | 10 | 300 | 100/100 | ○ |
| comparative example5 | No | don't | 20 | 500 | 130/130 | Δ |

**(Table 2)**

| | fiber diameter (µ m) | Area Weight (g/cm²) | static friction coefficient | Stiffness(mm) | | | feeling | Post Processability |
|---|---|---|---|---|---|---|---|---|
| | | | | Longitudinal | Transverse | sum | | |
| example3 | 18 | 20 | 0.18 | 53 | 37 | 90 | ⓞ | ⓞ |
| example4 | 18 | 20 | 0.28 | 59 | 38 | 97 | ○ | ⓞ |
| comparative example1 | 18 | 20 | 0.65 | 64 | 39 | 103 | Δ | ⓞ |
| comparative example2 | 18 | 20 | 0.45 | 60 | 38 | 98 | Δ | ⓞ |
| comparative example3 | 18 | 18 | 0.62 | 57 | 38 | 95 | Δ | ⓞ |
| comparative example4 | 18 | 20 | 0.15 | 39 | 31 | 65 | ⓞ | Δ |
| comparative example5 | 14 | 17 | 0.55 | 43 | 20 | 63 | ⓞ | Δ |

Further, the relation between the coefficient of static friction and stiffness of spunbonded nonwoven fabric of examples of the present invention and comprative examples is shown in Fig. 1. It is clear that spunbonded nonwoven fabric of the present invention (in the range of dotted line) is different markedly from conventional nonwoven fabric.

### Industrial Availability

The present invention is usable as the nonwoven polyolefin resin fabric and the absorbent article used this nonwoven fabric, and it is especially favorable in flexibility, feeling, touch to the skin, and is excellent in strength, bending rigidity and post processability, and it is usable suitably to various use, especially as the material for the absorbent article such as disposable diaper and the like.

## Claims

1. A spunbonded nonwoven fabric which comprises a spunbonded nonwoven fabric made of a polyolefin resin and has an average fiber diameter of 5 to 60 µm, area weight of 5 to 200 g/m² and a coefficient of static friction of 0.1 to 0.4.

2. The spunbonded nonwoven fabric according to claims 1, wherein the nonwoven fabric contains a lubricant.

3. The spunbonded nonwoven fabric according to claims 1 or 2, wherein the lubricant is a fatty acid amide compound, and its content is 0.05 to 1.0 mass %.

4. The spunbonded nonwoven fabric according to any one of claims 1 to 3, wherein the polyolefin resin is a polypropylene resin.

5. The spunbonded nonwoven fabric according to any one of claims 1 to 4, wherein the fiber is hydrophlicity imparting treated.

6. A spunbonded nonwoven fabric which comprises a spunbonded nonwoven polypropylene resin fabric having stiffness, which is the sum of the values for the longitudinal and transverse directions as obtained according to JIS L1096 6.19.1, method A that is 45° cantilever method, of 70 to 120 mm, and a coefficient of static friction of 0.1 to 0.4.

7. The spunbonded nonwoven fabric according to claim 6, wherein the nonwoven fabric has the average fiber diameter of 10 to 30 µm, and area weight of 10 to 30 g/m².

8. The spunbonded nonwoven fabric according to claims 6 or 7, wherein the nonwoven fabric contains the lubricant of 0.15 to 1.0 mass %.

9. The spunbonded nonwoven fabric according to claim 8, wherein the lubricant is a fatty acid amide compound.

10. The spunbonded nonwoven fabric according to any one of claims 6 to 9, wherein the fiber constituting of the nonwoven fabric is hydrophilicity imparting treated.

11. An absorbent article made by using the nonwoven fabric according to any one of claims 1 to 10.

12. The absorbent article according to claim 11, wherein the absorbent article is a disposable diaper, a sanitary napkin or incontinence pad.
